# EUROPEAN PATENT APPLICATION

(11) **EP 4 242 309 A1**
(43) Date of publication of application: **13.09.2023**
(21) Application number: 21889301.4
(22) Date of filing: 09.11.2021
(51) Int. Cl.: C12N 15/62, A61K 35/12, A61K 35/17, A61K 39/00, A61P 35/00, C07K 14/705, C07K 16/28, C07K 19/00, C12N 1/15, C12N 1/19, C12N 1/21, C12N 5/10, C12N 15/12, C12N 15/13

(54) **CHIMERIC ANTIGEN RECEPTOR**

(30) Priority: 09.11.2020 JP 2020186414
(71) Applicant: National University Corporation Tokai National Higher Education and Research System, Nagoya-shi, Aichi 464-8601 (JP)
(72) Inventor: TERAKURA, Seitaro, Nagoya-shi, Aichi 464-8601 (JP); JULAMANEE, Jakrawadee, Nagoya-shi, Aichi 464-8601 (JP); KIYOI, Hitoshi, Nagoya-shi, Aichi 464-8601 (JP)
(74) Representative: Hoffmann Eitle
(86) International application number: PCT/JP2021/041067
(87) International publication number: WO 2022/097750

(57) **Abstract**

An object of the present invention is to provide a novel chimeric antigen receptor. The object can be achieved by a chimeric antigen receptor, comprising an antigen-binding domain, a transmembrane domain, and an intracellular signal domain containing a CD79A intracellular domain and a CD40 intracellular domain,
wherein (A) the number of constituent amino acid residues between the antigen-binding domain and the transmembrane domain is 100 or less and/or (B) a target antigen of the antigen-binding domain is an antigen other than CD19.

## Description

### Technical Field

The present invention relates to a chimeric antigen receptor and the like.

### Background Art

A chimeric antigen receptor (hereinafter also referred to a "CAR") can bind to an antigen according to the specificity of the antibody, thereby causing target-cell cytotoxicity, cytokine release, and T-cell division after stimulation. Gene transfer of CAR can provide specificity to T cells, and cell preparation is generally easier than with culture amplification of endogenous tumor-specific T-cell receptor (TCR)-positive T cells. In addition, CAR-based therapy (CAR-T therapy) is superior to antibody therapy in that it exhibits higher cytotoxic activity and does not require multiple treatments because autonomous amplification occurs by antigen stimulation after a single transfusion. TCR gene transfer targets a complex of HLA and a peptide presented on the HLA, and thus, treatment can be performed only when a patient has a specific HLA (HLA restriction). CARs have a greater advantage in that they are not restricted by HLA. Specifically, if the target antigen in a tumor cell is positive, it is possible to provide treatment for all patients who are positive for the target antigen.

In chimeric antigen receptor gene-transfected T-cell (CAR-T cell) therapy using CD19CAR-T has been approved as a drug in the United States. Its clinical efficacy is high, and it is expected to be clinically useful for various malignant tumors (see, e.g., Patent Literature 1).

### Citation List

### Patent Literature

PTL 1: Japanese Patent No. 5312721

### Summary of Invention

### Technical Problem

An object of the present invention is to provide a novel chimeric antigen receptor. Preferably, an object of the present invention is to provide an antigen receptor that is more effective in preventing or treating cancer.

### Solution to Problem

The present inventor conducted extensive research in light of the above object and found that it is preferable to use an intracellular signal domain containing a CD79A intracellular domain and a CD40 intracellular domain. As a result of further research, the inventor found that the object can be attained by the following as one embodiment: a chimeric antigen receptor comprising an antigen-binding domain, a transmembrane domain, and an intracellular signal domain containing a CD79A intracellular domain and a CD40 intracellular domain, wherein (A) the number of constituent amino acid residues between the antigen-binding domain and the transmembrane domain is 100 or less and/or (B) a target antigen of the antigen-binding domain is an antigen other than CD19. The present inventor conducted further research based on this finding, and as a result, the present invention was accomplished. Specifically, the present invention includes the following embodiments.

Specifically, the present invention includes the following embodiments.
1. A chimeric antigen receptor, comprising an antigen-binding domain, a transmembrane domain, and an intracellular signal domain containing a CD79A intracellular domain and a CD40 intracellular domain, wherein (A) the number of constituent amino acid residues between the antigen-binding domain and the transmembrane domain is 100 or less and/or (B) a target antigen of the antigen-binding domain is an antigen other than CD19.
2. The chimeric antigen receptor according to Item 1, wherein the intracellular signal domain contains an intracellular activation domain.
3. The chimeric antigen receptor according to Item 2, wherein the intracellular activation domain is at least one member selected from the group consisting of a CD3ζ intracellular domain and a FcεRIγ intracellular domain.
4. The chimeric antigen receptor according to Item 2 or 3, wherein the CD79A intracellular domain, the CD40 intracellular domain, and the intracellular activation domain are arranged in this order from the transmembrane domain side.
5. The chimeric antigen receptor according to any one of Items 1 to 4, comprising a hinge region of IgG or a portion thereof between the antigen-binding domain and the transmembrane domain.
6. The chimeric antigen receptor according to any one of Items 1 to 5, wherein the number of the constituent amino acid residues is 1 to 30.
7. The chimeric antigen receptor according to any one of Items 1 to 6, wherein the structure of the antigen-binding domain is a single-chain antibody structure.
8. The chimeric antigen receptor according to any one of Items 1 to 7, wherein the target antigen of the antigen-binding domain is at least one member selected from the group consisting of CD19, GD2, GD3, CD20, CD37, CEA, HER2, EGFR, type III mutant EGFR, CD38, BCMA, MUC-1, PSMA, WT1, cancer testis antigens, mutation peptides, hTERT, PRAME, TYRP1, mesothelin, PMEL, and mucin.
9. A polynucleotide encoding the antigen receptor according to any one of Items 1 to 8.
10. A cell comprising the polynucleotide according to Item 9.
11. The cell according to Item 10, which is a lymphocyte cell.
12. A pharmaceutical composition comprising the cell according to Item 10 or 11.
13. The pharmaceutical composition according to Item 12, which is for use in the treatment, prevention, or improvement of cancer.

### Advantageous Effects of Invention

The present invention provides a novel antigen receptor. The use of the antigen receptor can effectively treat or prevent cancer.

### Brief Description of Drawings

Fig. 1 shows a schematic diagram of the structure of part of the CAR constructs prepared in Test Example 1.
Fig. 2 shows bioluminescence imaging results of Test Example 3. The vertical axis shows the luminescence intensity (the degree of amount of cancer). The horizontal axis shows the number of days elapsed after inoculation with cancer cells. Data are presented as mean ± SEM (two-way ANOVA;*P < 0.05, **P < 0.01) and were calculated from three independent experiments using three different donors (CD19 CAR SH T cells: n = 8 per group; tEGFR-T: n = 4).
Fig. 3 shows survival curves of Test Example 3. The vertical axis shows the survival rate of mice. The horizontal axis shows the number of days elapsed after inoculation with cancer cells. Wilcoxon test; *P < 0.05, ****P < 0.0001.
Fig. 4 shows bioluminescence imaging results of Test Example 4 (in the case of short-hinge constructs). The vertical axis shows the luminescence intensity (the degree of amount of cancer). The horizontal axis shows the number of days elapsed after inoculation with cancer cells. Data are presented as mean ± SEM (two-way ANOVA; *P < 0.05, **P < 0.01, ***P < 0.001, ****P < 0.0001) and were calculated from three independent experiments using three different donors (CD19 CAR SH T cells: n = 13 per group; tEGFR-T: n = 7).
Fig. 5 shows bioluminescence imaging results of Test Example 4 (in the case of long-hinge constructs). The vertical axis shows the luminescence intensity (the degree of amount of cancer). The horizontal axis shows the number of days elapsed after inoculation with cancer cells. Data are presented as mean ± SEM (two-way ANOVA; ***P < 0.001) and were calculated from two independent experiments using two different donors (CD19 CAR LH T cells: n = 8 per group; tEGFR-T: n = 6).
Fig. 6 shows survival curves of Test Example 4 (in the case of short-hinge constructs). The vertical axis shows the survival rate of mice. The horizontal axis shows the number of days elapsed after inoculation with cancer cells.
Fig. 7 shows survival curves of Test Example 4 (in the case of long-hinge constructs). The vertical axis shows the survival rate of mice. The horizontal axis shows the number of days elapsed after inoculation with cancer cells.
Fig. 8 shows the results of measuring the number of viable cells in Test Example 5. The vertical axis shows the relative number of viable CD19 CAR-T cells, and the horizontal axis shows the number of days elapsed after the start of co-culture. The left graph shows the results when IL-2 was not added, and the right graph shows the results when IL-2 was added.
Fig. 9 shows the results of measuring the number of viable cells in Test Example 6. The vertical axis shows the relative number of viable CD37 CAR-T cells, and the horizontal axis shows the number of days elapsed after the start of co-culture.
Fig. 10 shows the results of measuring the number of viable cells in Test Example 7. The vertical axis shows the relative number of viable CD20 CAR-T cells, and the horizontal axis shows the number of days elapsed after the start of co-culture.

### Description of Embodiments

### 1. Definitions

In the present specification, the terms "comprising," "containing," and "including" include the concepts of comprising, containing, consisting essentially of, and consisting of.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S, Altschul SF. Methods for assessing the statistical significance of molecular sequence features by using general scoring schemes, Proc Natl Acad Sci USA. 87: 2264-2268 (1990), Karlin S, Altschul SF. Applications and statistics for multiple high-scoring segments in molecular sequences, Proc Natl Acad Sci USA. 90: 5873-7 (1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/). The "identity" of base sequences is also defined in the same manner as above.

In the present specification, "conservative substitution" means the substitution of an amino acid residue with an amino acid residue having a similar side chain. For example, the substitution between amino acid residues having a basic side chain, such as lysine, arginine, or histidine, is considered to be a conservative substitution. The following substitutions between other amino acid residues are also considered to be a conservative substitution: the substitution between amino acid residues having an acidic side chain, such as aspartic acid and glutamic acid; the substitution between amino acid residues having an uncharged polar side chain, such as glycine, asparagine, glutamine, serine, threonine, tyrosine, or cysteine; the substitution between amino acid residues having a nonpolar side chain, such as alanine, valine, leucine, isoleucine, proline, phenylalanine, methionine, or tryptophan; the substitution between amino acid residues having a β-branched side chain, such as threonine, valine, or isoleucine; and the substitution between amino acid residues having an aromatic side chain, such as tyrosine, phenylalanine, tryptophan, or histidine.

In the present specification, "CDR" is an abbreviation for complementarity determining region. CDR is a region in the variable regions of immunoglobulins or T-cell receptors and is deeply involved in the specific binding of an antibody or T-cell receptor for an antigen. The phrase "light-chain CDR" refers to a CDR present in the light-chain variable regions of immunoglobulins, and the phrase "heavy-chain CDR" refers to a CDR present in the heavy-chain variable regions of immunoglobulins. In T-cell receptors, there are also α chain, β chain, γ chain, δ chain, and like chains, and the same applies to the CDRs of these chains.

In the present specification, the phrase "variable region" refers to a region containing CDR1 to CDR3 (simply "CDRs 1-3" below). The order in which CDRs 1-3 are arranged is not particularly limited; however, the variable region preferably refers to a region in which CDR1, CDR2, and CDR3 are arranged in this order in the direction from the N-terminus toward the C-terminus or in the reverse order either consecutively or via other amino acid sequences referred to as "framework regions" (FRs), which are described later. The phrase "heavy-chain variable region" refers to a region of immunoglobulins in which heavy-chain CDRs 1-3 are arranged, and the phrase "light-chain variable region" refers to a region of immunoglobulins in which light-chain CDRs 1-3 are arranged. In T-cell receptors, there are also α chain, β chain, γ chain, δ chain, and like chains, and the same applies to the CDRs of these chains.

The regions other than CDRs 1-3 of each variable region are referred to as "framework regions" (FRs), as mentioned above. In particular, the region between the N-terminus and CDR1 of a variable region is defined as FR1, the region between CDR1 and CDR2 as FR2, the region between CDR2 and CDR3 as FR3, and the region between CDR3 and the C-terminus of a variable region as FR4 .

### 2. Chimeric Antigen Receptor

In one embodiment, the present invention relates to a chimeric antigen receptor, comprising an antigen-binding domain, a transmembrane domain, and an intracellular signal domain containing a CD79A intracellular domain and a CD40 intracellular domain, wherein (A) the number of constituent amino acid residues between the antigen-binding domain and the transmembrane domain is 100 or less and/or (B) a target antigen of the antigen-binding domain is an antigen other than CD19 (in this specification referred to as "the chimeric antigen receptor of the present invention"). The chimeric antigen receptor of the present invention is described below.

In one embodiment, the present invention relates to a chimeric antigen receptor comprising an antigen-binding domain, a transmembrane domain, and an intracellular signal domain containing a CD79A intracellular domain and a CD40 intracellular domain. The chimeric antigen receptor is described in accordance with the description of the chimeric antigen receptor of the present invention.

The antigen-binding domain is not particularly limited as long as the domain is positioned extracellularly when the chimeric antigen receptor of the present invention is placed on the cell membrane, and the domain can recognize an antigen and bind to the antigen. Examples of the antigen include CD19, GD2, GD3, CD20, CD37, CEA, HER2, EGFR, type III mutant EGFR, CD38, BCMA, MUC-1, PSMA, WT1, cancer testis antigens (e.g., NY-ESO-1 and MAGE-A4), mutation peptides (e.g., k-ras, h-ras, and p53), hTERT, PRAME, TYRP1, mesothelin, PMEL, and mucin; and complexes of these fragments with MHC (pMHC). Of these, in one embodiment of the present invention, the antigen is preferably CD19. In another embodiment of the present invention, the antigen is preferably an antigen other than CD19, and more preferably CD20 and CD37. The antigen-binding domain typically contains one or more, preferably two, three, four, or five or more CDRs, or more preferably all of the six CDRs of an antibody or T-cell receptor for an antigen (e.g., in the case of CDRs of antibodies, heavy-chain CDR1, heavy-chain CDR2, heavy-chain CDR3, light-chain CDR1, light-chain CDR2, and light-chain CDR3). The antigen-binding region more preferably contains a variable region of an antibody or T-cell receptor for an antigen (in the case of antibodies, for example, the heavy-chain variable regions and/or light-chain variable regions.)

The antigen-binding domain preferably has a single-chain antibody structure, and more preferably has the structure of scFv. When a heavy-chain variable region and a light-chain variable region are contained as in the scFv structure, the heavy-chain variable region and the light-chain variable region are typically linked via a linker. Any linker can be used as long as antigen bindability is not significantly impaired. The linker is preferably a linker of glycine or a linker composed of glycine and serine (e.g., GGS linker, GS linker, and GGG linker). The length of the linker is not particularly limited. The number of amino acid residues in the linker is, for example, 5 to 30, and preferably 10 to 25. Arrangement of the heavy-chain variable region and the light-chain variable region is not particularly limited. The light-chain variable region may be at a position closer to the N-terminus, or the heavy-chain variable region may be at a position closer to the N-terminus. The arrangement is preferably the former.

The transmembrane domain is not particularly limited as long as the domain is placed in a cell when the chimeric antigen receptor of the present invention is placed on the cell membrane, and the domain can compose the chimeric antigen receptor. Examples of the transmembrane domain include transmembrane regions of CD28, CD3ε, CD8α, CD3, CD4, and CD4-1BB. The transmembrane region of each factor is known or can be easily determined from known sequence information (e.g., using a program to predict a transmembrane region). Alternatively, a transmembrane domain comprising an artificially constructed polypeptide may be used. These transmembrane domains may be suitably mutated as long as the function of the chimeric antigen receptor is not significantly inhibited.

As a preferable example of the transmembrane domain, a transmembrane region of CD28 is preferably used. A specific example of the transmembrane region of CD28 is an amino acid sequence described in (a) below or an amino acid sequence described in (b) below:
(a) an amino acid sequence represented by SEQ ID NO: 3, or
(b) an amino acid sequence that has at least 85% identity with the amino acid sequence represented by SEQ ID NO: 3, and that can be placed in a cell when the chimeric antigen receptor of the present invention is placed on the cell membrane.

In item (b) above, the identity is preferably at least 90%, more preferably at least 95%, still more preferably at least 98%, and particularly preferably at least 99%.

An example of the amino acid sequence described in item (b) is the following:
(b') an amino acid sequence that has the substitution, deletion, addition, or insertion of one or multiple amino acids in the amino acid sequence represented by SEQ ID NO: 3, and that can be placed in a cell when the chimeric antigen receptor of the present invention is placed on the cell membrane.

In item (b') above, "multiple amino acids" means, for example, 2 to 5 amino acids, preferably 2 to 3 amino acids, and more preferably 2 amino acids.

In one embodiment of the present invention, the antigen-binding domain and the transmembrane domain are preferably linked directly or via a spacer with a relatively short length. In the chimeric antigen receptor of the present invention, the number of constituent amino acid residues between the antigen-binding domain and the transmembrane domain can be, for example, 400 or less, 300 or less, or 250 or less, and particularly preferably 100 or less. The number of constituent amino acid residues is preferably 0 to 60, more preferably 0 to 40, and even more preferably 0 to 30. In a particularly preferable embodiment of the present invention, the number of amino acid residues is 1 or more, 4 or more, or 8 or more, and 25 or less, 20 or less, or 15 or less, and 1 to 25, 4 to 20, or 8 to 15. The sequence of the spacer is not particularly limited; for example, the sequence of the hinge region of IgG or portion thereof, preferably the hinge region of human IgG (e.g., subtype IgG1 or IgG4) or portion thereof, the hinge region and a portion of CH2, or a portion of a factor used for the transmembrane domain (e.g., CD28), etc. can be used as a spacer. It can be expected that the use of the sequence of the hinge region or a portion thereof will form a flexible spacer.

The intracellular signal domain is a domain that is placed in a cell when the chimeric antigen receptor of the present invention is placed on the cell membrane, and is capable of transmitting a signal necessary for exerting an effector function of an immune cell, specifically, transmitting a signal necessary for activation of an immune cell when the antigen-binding domain binds to an antigen. In the chimeric antigen receptor of the present invention, the intracellular signal domain contains a CD79A intracellular domain and a CD40 intracellular domain.

The CD79A intercellular domain can be any domain containing the amino acid sequence of the intracellular domain of amino acids of CD79A.

CD79A for use can be various types of CD79A derived from mammals, such as humans, monkeys, mice, rats, dogs, cats, and rabbits. Of these, CD79A derived from a target organism (e.g., humans) is preferable.

The amino acid sequences of CD79A intracellular domains derived from various organisms are known. Specifically, examples include the amino acid sequence represented by SEQ ID NO: 4.

The CD79A intracellular domain may be mutated, for example, by the substitution, deletion, addition, or insertion of amino acids as long as the CD79A intracellular domain has an NFκB signal-activating effect. The mutation is preferably a substitution, and more preferably a conservative substitution, from the standpoint of the lower likelihood of the CD79A intracellular domain losing the above effect.

A preferable specific example of the amino acid sequence of the CD79A intracellular domain is at least one member selected from the group consisting of the amino acid sequence described in (c) below and the amino acid sequence described in (d) below:
(c) an amino acid sequence represented by SEQ ID NO: 4, and
(d) an amino acid sequence that has at least 85% identity with the amino acid sequence represented by SEQ ID NO: 4, and that constitutes a protein having an NF_{K}B signal-activating effect.

In item (d) above, the identity is preferably at least 90%, more preferably at least 95%, still more preferably at least 98%, and particularly preferably at least 99%.

An example of the amino acid sequence described in item (d) is the following:
(d') an amino acid sequence that has the substitution, deletion, addition, or insertion of one or multiple amino acids in the amino acid sequence represented by SEQ ID NO: 4, and that constitutes a protein having an NF_{K}B signal-activating effect.

In item (d') above, "multiple amino acids" means, for example, 2 to 5 amino acids, preferably 2 to 3 amino acids, and more preferably 2 amino acids.

The CD40 intercellular domain can be any domain containing the amino acid sequence of the intracellular domain of amino acids of CD40.

CD40 for use can be various types of CD40 derived from mammals, such as humans, monkeys, mice, rats, dogs, cats, and rabbits. Of these, CD40 derived from a target organism (e.g., humans) is preferable.

The amino acid sequences of CD40 intracellular domains derived from various organisms are known. Specifically, examples include the amino acid sequence represented by SEQ ID NO: 5.

The CD40 intracellular domain may be mutated, for example, by the substitution, deletion, addition, or insertion of amino acids as long as the CD40 intracellular domain has an NF_{K}B signal-activating effect. The mutation is preferably a substitution, and more preferably a conservative substitution, from the standpoint of the lower likelihood of the CD40 intracellular domain losing its effect.

A preferable specific example of the amino acid sequence of the CD40 intracellular domain is at least one member selected from the group consisting of the amino acid sequence described in (e) below and the amino acid sequence described in (f) below:
(e) an amino acid sequence represented by SEQ ID NO: 5, and
(f) an amino acid sequence that has at least 85% identity with an amino acid sequence represented by SEQ ID NO: 5, and that constitutes a protein having an NF_{K}B signal-activating effect.

In item (f) above, the identity is preferably at least 90%, more preferably at least 95%, still more preferably at least 98%, and particularly preferably at least 99%.

An example of the amino acid sequence described in item (f) is the following:
(f') an amino acid sequence that has the substitution, deletion, addition, or insertion of one or multiple amino acids in the amino acid sequence represented by SEQ ID NO: 5, and that has an NF_{K}B signal-activating effect.

In item (f') above, "multiple amino acids" means, for example, 2 to 5 amino acids, preferably 2 to 3 amino acids, and more preferably 2 amino acids.

Arrangement of the CD79A intracellular domain and the CD40 intracellular domain is not particularly limited. The CD79A intracellular domain may be at a position closer to the N-terminus, or the CD40 intracellular domain may be at a position closer to the N-terminus. The arrangement is preferably the former. The CD79A intracellular domain and the CD40 intracellular domain are preferably directly linked or linked via a linker. Any linker can be used as long as an NF_{K}B signal-activating effect is not significantly impaired. The linker is preferably a linker of glycine or a linker composed of glycine and serine (GGS linker, GS linker, GGG linker, etc.). The length of the linker is not particularly limited. The number of amino acid residues in the linker is, for example, 1 to 20, preferably 1 to 10, more preferably 1 to 5.

The intracellular signal domain preferably comprises an intracellular activation domain. As the intracellular activation domain, for example, an intracellular domain, such as FcεRIγ in addition to CD3ζ, can be used. Preferable examples include CD3ζ. CD3 may be mutated as appropriate, as long as the functionality of the antigen receptor is not interfered with. Mutation of CD3 is preferably made such that CD3 contains ITAM (immunoreceptor tyrosine-based activation motif).

A preferable specific example of the intracellular activation domain is an amino acid sequence described in (g) below or an amino acid sequence described in (h) below:
(g) an amino acid sequence represented by SEQ ID NO: 6, or
(h) an amino acid sequence that has at least 85% identity with the amino acid sequence represented by SEQ ID NO: 6, and that has an immune cell activation effect.

In item (h) above, the identity is preferably at least 90%, more preferably at least 95%, still more preferably at least 98%, and particularly preferably at least 99%.

An example of the amino acid sequence described in item (h) is the following:
(h') an amino acid sequence that has the substitution, deletion, addition, or insertion of one or multiple amino acids in the amino acid sequence represented by SEQ ID NO: 6, and that has an immune cell activation effect.

In item (h') above, "multiple amino acids" means, for example, 2 to 15 amino acids, preferably 2 to 10 amino acids, more preferably 2 to 5 amino acids, and even more preferably 2 to 3 amino acids.

The position of the intracellular activation domain is not particularly limited. The intracellular activation domain is preferably at a position closer to the intracellular side (opposite the transmembrane domain) than the CD79A intracellular domain and the CD40 intracellular domain. In a particularly preferred embodiment of the present invention, the CD79A intracellular domain, the CD40 intracellular domain, and the intracellular activation domain are arranged in this order from the transmembrane domain side.

The chimeric antigen receptor of the present invention may comprise other regions than those described above. Other regions include, for example, a costimulatory intracellular domain, a leader sequence (signal peptide) to facilitate transport of the CAR onto the cell membrane (for example, the leader sequence of a GM-CSF receptor), spacer/linker (for example, between the transmembrane region and the intracellular signal domain, between each domain within the intracellular signal domain), and the like. The intracellular domain of a co-stimulator can be of any intracellular domain derived from a co-stimulator of cells such as T cells. For example, at least one member selected from the group consisting of OX40, 4-1BB, GITR, CD27, CD278, CD28, and the like can be suitably selected and used.

There have been several reports of experiments, clinical studies, and the like using CARs (see, e.g., Rossig C et al. Mol Ther 10: 5-18, 2004; Dotti G et al. Hum Gene Ther 20: 1229-1239, 2009; Ngo MC et al. Hum Mol Genet 20 (R1): R93-99, 2011; Ahmad N et al. Mol Ther 17: 1779-1787, 2009; Pule M A et al. Nat Med 14: 1264-1270, 2008; Louis CU et al. Blood 118: 6050-6056, 2011; Kochenderfer J N et al. Blood 116: 4099-4102, 2010; Kochenderfer J N et al. Blood 119: 2709-2720, 2012; N Engl J Med 365: 725-733, 2011; Kalos M et al. Sci Transl Med 3: 95ra73, 2011; Brentjens R J et al. Blood 118: 4817-4828, 2011; Brentjens R J et al. Sci Transl Med 5: 177ra38, 2013). The chimeric antigen receptor of the present invention can be constructed with reference to these reports.

One particularly preferred embodiment of the chimeric antigen receptor of the present invention is a chimeric antigen receptor comprising a core region in which an antigen-binding domain, a transmembrane domain, a CD79A (CD40) intracellular domain and a CD40 (CD79A) intracellular domain, and an intracellular activation domain are arranged in this order. In the core region, the antigen-binding domain and the transmembrane domain are linked via a spacer; and the transmembrane domain and the CD79A (CD40) intracellular domain and the CD40 (CD79A) intracellular domain, the CD79A (CD40) intracellular domain and the CD40 (CD79A) intracellular domain, and the CD79A (CD40) intracellular domain and the CD40 (CD79A) intracellular domain and the intracellular activation domain are linked directly or via a spacer/linker. The spacer/linker is the same as described above.

The chimeric antigen receptor of the present invention may be a molecule formed of a single type of polypeptide or a molecule formed of a complex of two or more types of polypeptides. The chimeric antigen receptor of the present invention may also be a molecule formed of a polypeptide or of a complex of polypeptides, or a molecule formed of a polypeptide or complex of polypeptides to which another substance (e.g., a fluorescent substance, a radioactive substance, or an inorganic particle) is linked.

The chimeric antigen receptor of the present invention may be chemically modified. The polypeptide that constitutes the chimeric antigen receptor of the present invention may have a carboxyl group (-COOH), carboxylate (-COO-), amide (-CONH₂), or ester (-COOR) at the C-terminus. "R" in the ester is, for example, a C₁₋₆ alkyl group, such as methyl, ethyl, n-propyl, isopropyl, or n-butyl; a C₃₋₈ cycloalkyl group, such as cyclopentyl or cyclohexyl; a C₆₋₁₂ aryl group, such as phenyl or α-naphthyl; a phenyl-C₁₋₂ alkyl group, such as benzyl or phenethyl; a C₇₋₁₄ aralkyl group such as an α-naphthyl-C₁₋₂ alkyl group such as α-naphthyl methyl; or a pivaloyloxymethyl group. The polypeptide that constitutes the chimeric antigen receptor of the present invention may have an amidated or esterified carboxyl group (or carboxylate), which is not the carboxyl group at the C-terminus. The ester in this case may be, for example, the esters of the C-terminus described above. The polypeptide that constitutes the chimeric antigen receptor of the present invention further includes polypeptides having the amino group of the N-terminus amino acid residue protected by a protective group (e.g., a C₁₋₆ acyl group, including a C₁₋₆ alkanoyl, such as a formyl group and an acetyl group), polypeptides having the N-terminal glutamine residue pyroglutamated that can be formed due to cleavage in vivo; and polypeptides having a substituent (e.g., -OH, -SH, an amino group, an imidazole group, an indole group, and a guanidino group) on a side change of an amino acid in the molecule protected by an appropriate protective group (e.g., a C₁₋₆ acyl group, including a C₁₋₆ alkanoyl group, such as a formyl group and an acetyl group).

The chimeric antigen receptor of the present invention may have a protein or peptide (e.g., a known protein tag or signal sequence) added. Examples of protein tags include biotin, a His tag, a FLAG tag, a Halo tag, a MBP tag, a HA tag, a Myc tag, a V5 tag, a PA tag, and a fluorescent protein tag.

The chimeric antigen receptor of the present invention may be a pharmaceutically acceptable salt formed with an acid or base. The salt can be any pharmaceutically acceptable salt, and can be either an acid salt or a basic salt. Examples of acid salts include inorganic acid salts, such as hydrochloride, hydrobromide, sulfate, nitrate, and phosphate; organic acid salts, such as acetate, propionate, tartarate, fumarate, maleate, malate, citrate, methanesulfonate, and para-toluenesulfonate; and amino acid salts, such as aspartate and glutamate. Examples of basic salts include alkali metal salts, such as sodium salts and potassium salts; and alkaline-earth metal salts, such as calcium salts and magnesium salts.

The chimeric antigen receptor of the present invention may be in the form of a solvate. The solvent can be any pharmaceutically acceptable solvent, and may be, for example, water, ethanol, glycerol, or acetic acid.

The techniques for producing a chimeric antigen receptor and a CAR-T cell that expresses the chimeric antigen receptor are known. Chimeric antigen receptors and CAR-T cells can be produced in accordance with a known method or an equivalent method.

### 3. Polynucleotide and Use Thereof

In an embodiment, the present invention relates to a polynucleotide encoding the chimeric antigen receptor of the present invention (sometimes referred to as "the polynucleotide of the present invention" in the present specification.) The polynucleotide of the present invention is described below.

The polynucleotide of the present invention may contain other sequences in addition to the coding sequence of the chimeric antigen receptor of the present invention. Preferably, the polynucleotide of the present invention expressibly contains the sequence of the chimeric antigen receptor of the present invention. Other sequences include promoter sequences, enhancer sequences, repressor sequences, insulator sequences, origins of replication, reporter protein (e.g., fluorescent proteins) coding sequences, and drug-resistant-gene-coding sequences.

The polynucleotide of the present invention preferably contains an expression cassette of the chimeric antigen receptor of the present invention. The expression cassette comprises a promoter and the coding sequence of the chimeric antigen receptor of the present invention, which is under the control of the promoter. The coding sequence is typically placed downstream of the promoter so as to be under the control of the promoter. Examples of promoters that can be used for the CAR expression cassette include CMV-IE (cytomegalovirus early gene-derived promoter), SV40ori, retroviral LTP, SRα, EF1α, and β-actin promoters. The promoter is operably linked to the coding sequence. Here, "the promoter is operably linked to the coding sequence" has the same meaning as "the coding sequence is placed under the control of the promoter," and typically, the coding sequence is linked to the 3' terminal side of the promoter directly or via another sequence. A poly-A additional signal sequence is placed downstream of the coding sequence. Transcription is terminated by the use of the poly-A additional signal sequence. The poly-A additional signal sequence may be, for example, the poly-A additional sequence of SV40, the poly-A additional sequence of a bovine growth hormone gene, or the like.

The expression cassette may contain a gene for detection (a reporter gene, cell-specific or tissue-specific gene, selection marker gene, etc.), an enhancer sequence, a WRPE sequence, or the like. The gene for detection is used for the judgement of success or failure or efficiency of the introduction of the expression cassette, detection of CAR gene expression or judgement of the expression efficiency, or selection and collection of cells in which the CAR gene is expressed. On the other hand, the use of an enhancer sequence improves expression efficiency. Usable examples of the gene for detection include a neo gene imparting resistance to neomycin, an npt gene (Herrera Estrella, EMBO J. 2 (1983), 987-995) and an nptII gene (Messing & Viera. Gene 1 9:259-268 (1982)) imparting resistance to kanamycin, a hph gene imparting resistance to hygromycin (Blochinger & Digglmann, Mol Cell Bio 4:2929-2931), and a dhfr gene imparting resistance to methotrexate (Bourouis et al., EMBO J. 2(7)) (the above are marker genes); genes of fluorescence proteins, such as a luciferase gene (Giacomin, P1. Sci. 116 (1996), 59-72; Scikantha, J. Bact. 178 (1996), 121), and a β-glucuronidase (GUS) gene, GFP (Gerdes, FEBS Lett. 389 (1996), 44-47) and their variants (EGFP, d2EGFP, etc.) (the above are reporter genes); and an epidermal growth factor receptor (EGFR) gene that lacks an intracellular domain. The gene for detection is linked to a CAR gene, for example, via a bicistronic control sequence (for example, internal ribosome entry site (IRES)) and a sequence encoding a self-cleaving peptide. Examples of the self-cleaving peptide include, but are not limited to, the 2A peptide (T2A) derived from *Thosea asigna* virus. Known examples of the self-cleaving peptide include the 2A peptide (F2A) derived from foot-and-mouth disease virus (FMDV), 2A peptide (E2A) derived from equine rhinitis A virus (ERAV), and 2A peptide (P2A) derived from *Porcine teschovirus* (PTV-1).

The polynucleotide of the present invention may be a linear polynucleotide or a cyclic polynucleotide (e.g., a vector). The vector may be a plasmid vector or a viral vector. The vector may be, for example, a vector for cloning or for expression. Examples of the vector for expression include vectors for prokaryotic cells, such as *Escherichia coli,* or actinomycetes, and vectors for eukaryotic cells, such as yeast cells, insect cells, or mammalian cells. More specifically, examples of viral vectors include retrovirus vectors, lentivirus vectors, adenovirus vectors, adeno-associated virus vectors, herpesvirus vectors, and Sendai virus vectors; and examples of non-viral vectors include various plasmid vectors, liposome vectors, positively charged liposome vectors (Felgner, P.L., Gadek, T.R., Holm, M., et al. Proc. Natl. Acad. Sci., 84:7413-7417, 1987), YAC vectors, and BAC vectors.

The polynucleotide of the present invention includes not only DNA and RNA, but also known chemically modified DNA or RNA as described below. To prevent degradation by hydrolases, such as nucleases, the phosphate residue (phosphate) of each nucleotide can be substituted with, for example, a chemically modified phosphate residue, such as phosphorothioate (PS), methylphosphonate, or phosphorodithionate. The hydroxyl group at position 2 of a sugar (ribose) of each ribonucleotide may be, for example, replaced with -OR (R represents, for example, CH3 (2'-O-Me), CH₂CH₂OCH₃ (2'-O-MOE), CH₂CH₂NHC (NH) NH₂, CH₂CONHCH₃, or CH₂CH₂CN). Additionally, the base moiety (pyrimidine, purine) may be chemically modified, by, for example, introduction of a methyl group or a cationic functional group into position 5 of the pyrimidine base, or substitution of the carbonyl group at position 2 with thiocarbonyl. Additionally, the polynucleotide of the present invention also includes, but is not limited to, those in which the phosphoric acid moiety or hydroxyl moiety is modified with biotin, an amino group, a lower alkyl amine group, an acetyl group, or the like. The term "polynucleotide" includes not only natural nucleic acids but also BNA (bridged nucleic acid), LNA (locked nucleic acid), and PNA (peptide nucleic acid).

A cell having an anticancer effect can be obtained by a method comprising introducing the polynucleotide of the present invention into cells. The cells express the chimeric antigen receptor of the present invention. One such embodiment is explained below.

Examples of cells (cells for CAR expression) into which the polynucleotide of the present invention is introduced include CD4 positive CD8 negative T cells, CD4 negative CD8 positive T cells, T cells prepared from iPS cells, αβ-T cells, γδ-T cells, NK cells, and NKT cells. Various cell populations can be used as long as they contain lymphocytes or progenitor cells as described above. PBMCs (peripheral blood mononuclear cells) collected from peripheral blood are some of the preferred target cells. Specifically, in a preferred embodiment, a gene transfer operation is performed on PBMCs. PBMCs can be prepared according to or in accordance with an ordinary method.

It is preferable to activate the cells for CAR expression before the introduction of the polynucleotide of the present invention. For example, the CAR expression cells can be activated by stimulation with an anti-CD3 antibody and an anti-CD 28 antibody. For example, by culturing in a culture container (for example, a culture dish) having a culture surface coated with an anti-CD3 antibody and an anti-CD28 antibody, stimulation with an anti-CD3 antibody and an anti-CD28 antibody can be applied. The stimulation can also be performed using magnetic beads coated with an anti-CD3 antibody and an anti-CD28 antibody (for example, Dynabeads T-Activator CD3/CD28 provided by Veritas Corporation.)

In order to increase the survival rate or proliferation rate of the cells, it is preferred to use a culture medium containing a T-cell growth factor in activation treatment. As the T-cell growth factor, IL-2, IL-15, IL-7, and the like can be used. T-cell growth factors such as IL-2, IL-15, and IL-7 can be prepared according to a conventional method. Commercially available products can also be used. The use of T-cell growth factors of animals other than humans is not excluded, but generally, T-cell growth factors derived from humans (which may be recombinant) are used.

Typically, for their application (administration to a patient), cells after introduction of the polynucleotide of the present invention (polynucleotide-introduced lymphocytes of the present invention) are propagated. For example, the polynucleotide-introduced lymphocytes of the present invention are cultured (subcultured as necessary) using a culture medium to which a T-cell growth factor has been added. In addition to this culture, the same treatment (reactivation) as in the case of activation of the CAR-expression cells may be performed.

Although a medium to which serum (human serum, fetal bovine serum, and the like) is added may be used for culturing the CAR expression cells and the polynucleotide-introduced lymphocytes of the present invention, by using a serum-free medium, it is possible to prepare cells having the advantages of a high degree of safety in clinical application and occurrence of a difference in culture efficiency due to a difference between serum lots being unlikely. When serum is used, it is preferable to use autologous serum, i.e., serum collected from a patient to whom CAR transgenic lymphocytes are administered.

### 4. Cell

In one embodiment, the present invention relates to a cell comprising the polynucleotide of the present invention (which may be referred to as "the cell of the present invention" in the present specification). The cell is described below.

Cells from which the cell of the present invention is derived are not particularly limited. For the purpose of using the cell of the present invention in the purification of the chimeric antigen receptor of the present invention, for example, cells that can be used for protein expression (e.g., insect cells, eukaryotic cells, mammal cells) can be used as the origin cells.

The cell of the present invention is preferably a lymphocyte cell (a T cell (e.g., a CD4-positive CD8-negative T cell, a CD4-negative CD8-positive T cell, a T cell prepared from iPS cells, αβ-T cells, γδ-T cells, etc.), a NK cells, a NKT cell, etc.). These cells are preferably cells expressing the chimeric antigen receptor of the present invention. In a more specific embodiment of these cells, the antigen receptor of the present invention is expressed on the cell membrane, and preferably expressed in such a state that the antigen-binding domain of the present invention is exposed outside the cell membrane.

A T cell or the like expressing the chimeric antigen receptor recognizes the antigen in the antigen-binding region, and then intracellularly transfers a recognition signal to activate a signal that induces cytotoxic activity. In conjunction with this, the cell can mount attacks against other cells or tissues expressing the antigen or exerts cytotoxic activity.

When a cell exhibiting such a function is a CTL, it is called a chimeric antigen receptor T cell (CAR-T cell). Cells that have potential to exhibit cytotoxic activity, such as NK cells, can also exert cytotoxic activity when the antigen-binding region binds to the antigen, as with the chimeric antigen receptor T-cell. Thus, a host cell comprising the polynucleotide encoding the chimeric antigen receptor of the present invention (in particular, a host cell having cytotoxic activity) is useful as an active ingredient of pharmaceutical compositions.

### 5. Pharmaceutical Composition

In one embodiment, the present invention relates to a pharmaceutical composition comprising the cell of the present invention (which may be referred to as "the pharmaceutical composition of the present invention" in the present specification). The pharmaceutical composition of the present invention is described below.

The pharmaceutical composition of the present invention can be widely used as a cell preparation for the treatment, prevention, or improvement of a tumor/cancer (target disease) expressing a target antigen of an antigen-binding domain. Target diseases include solid cancers and hematological cancers. Examples of the target disease include various B-cell malignant lymphomas (B-cell acute lymphocytic leukemia, follicular lymphoma, diffuse large-cell lymphoma, mantle cell lymphoma, MALT lymphoma, intravascular B-cell lymphoma, CD20 positive Hodgkin lymphoma), myeloproliferative diseases, myelodysplastic/myeloproliferative tumors (CMML, JMML, CML, MDS/MPN-UC), myelodysplastic syndrome, acute myeloid leukemia, multiple myeloma, lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, rhabdomyosarcoma, and the like. "Treatment" includes alleviation (moderation) of symptoms or associated symptoms characteristic to the target diseases, inhibition or retard of deterioration of symptoms. "Prevention" means prevention or retard of development/expression of diseases (disorders) or their symptoms, or decrease of the risk of development/expression. On the other hand, "improvement" means alleviation (moderation), change for the better, amelioration, or healing (containing partial healing).

The pharmaceutical composition of the present invention contains a therapeutically effective amount of the cells of the present invention. For example, 1 × 10⁴ to 1 × 10¹⁰ cells can be contained for one administration. The cell preparation may contain dimethylsulfoxide (DMSO) or serum albumin for the purpose of cell protection; antibiotics for the purpose of preventing bacterial contamination; and various components (for example, vitamins, cytokine, growth factors, and steroids) for the purpose of cell activation, proliferation, or inductive differentiation.

The administration route of the CAR transgenic lymphocytes or cell preparation of the present invention is not particularly limited. For example, it is administered by intravenous injection, intraarterial injection, portal vein injection, intradermal injection, subcutaneous injection, intramuscular injection, or intraperitoneal injection. Local administration may be used in place of systemic administration. Examples of the local administration include direct injection into target tissues, body parts, or organs. The administration schedule may be made according to the sex, age, body weight, and pathology of the subject (patient). A single dose or continuous or periodical multiple doses may be used.

### Examples

The present invention is described in detail below with reference to Examples. However, the present invention is not limited to these Examples.

### Test Example 1: Preparation of CAR Construct

### Test Example 1-1: Preparation of CD19.CD79a.40z.SH Construct

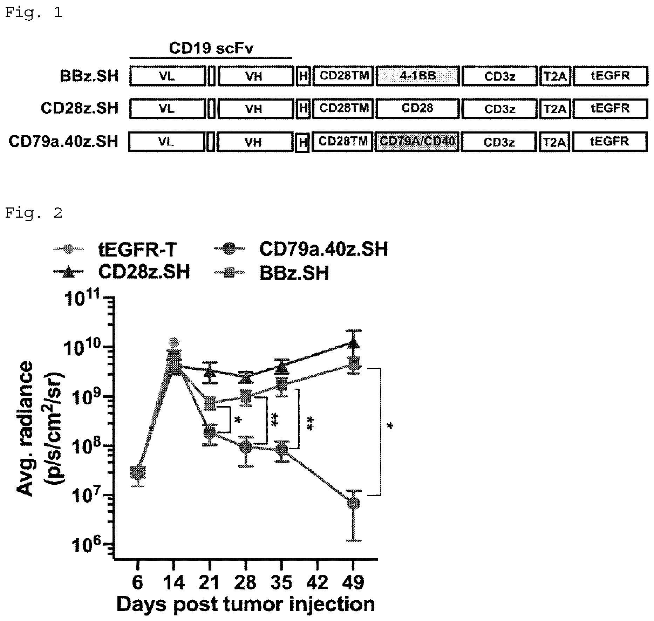

A CAR construct (Fig. 1; amino acid sequence: SEQ ID NO: 9; base sequence: SEQ ID NO: 18) comprising an anti-CD19 scFv domain (amino acid sequence: SEQ ID NO: 1; base sequence: SEQ ID NO: 10), an IgG4 hinge region (amino acid sequence: SEQ ID NO: 2; base sequence: SEQ ID NO: 11), a CD28 transmembrane domain (amino acid sequence: SEQ ID NO: 3, base sequence: SEQ ID NO: 12), a CD79A intracellular domain (amino acid sequence: SEQ ID NO: 4; base sequence: SEQ ID NO: 13), a GGG linker (amino acid sequence: N-terminus-GGG; base sequence: 5'-GGCGGAGGG), a CD40 intracellular domain (amino acid sequence: SEQ ID NO: 5; base sequence: SEQ ID NO: 14), a CD3ζ intracellular domain (amino acid sequence: SEQ ID NO: 6; base sequence: SEQ ID NO: 15), a T2A sequence (amino acid sequence: SEQ ID NO: 7; base sequence: SEQ ID NO: 16), a truncated EGFR domain (amino acid sequence: SEQ ID NO: 8; base sequence: SEQ ID NO: 17) arranged in this order from the N-terminus was designed, and the coding sequence of the CAR (base sequence: SEQ ID NO: 18) was introduced into an LZRS-pBMN-Z vector using restriction enzyme recognition sequences added to both ends thereof (HindIII on the 5'-side and NotI on the 3'-side).

### Test Example 1-2: Preparation of CD19.28z.SH Construct

In the same manner as in Test Example 1-1, a CAR construct (Fig. 1) that is the same as the CD79a.40z.SH construct except that a CD28 intracellular domain (amino acid sequence: SEQ ID NO: 19; base sequence: SEQ ID NO: 20) was used in place of the intracellular signal domain containing the CD79A intracellular domain, the GGG linker, and the CD40 intracellular domain was prepared.

### Test Example 1-3: Preparation of CD19.BBz.SH Construct

In the same manner as in Test Example 1-1, a CAR construct (Fig. 1) that is the same as the CD79a.40z.SH construct except that a 4-1BB intracellular domain (amino acid sequence: SEQ ID NO: 21; base sequence: SEQ ID NO: 22) was used in place of the intracellular signal domain containing the CD79A intracellular domain, the GGG linker, and the CD40 intracellular domain was prepared.

### Test Example 1-4: Preparation of CD19.CD79a.40z.LH Construct

In the same manner as in Test Example 1-1, a CAR construct that is the same as the CD19.CD79a.40z.SH construct except that an immunoglobulin hinge+CH2-CH3 region (amino acid sequence: SEQ ID NO: 23; base sequence: SEQ ID NO: 24) was used in place of the IgG4 hinge region was prepared.

### Test Example 1-5: Preparation of CD19.28z.LH Construct

In the same manner as in Test Example 1-1, a CAR construct that is the same as the CD19.28z.SH construct except that an immunoglobulin hinge+CH2-CH3 region (amino acid sequence: SEQ ID NO: 23; base sequence: SEQ ID NO: 24) was used in place of the IgG4 hinge region was prepared.

### Test Example 1-6: Preparation of CD37.CD79a.40z.SH Construct

In the same manner as in Test Example 1-1, a CAR construct that is the same as the CD19.CD79a.40z.SH construct except that an anti-CD37 scFv domain (amino acid sequence: SEQ ID NO: 25; base sequence: SEQ ID NO: 26) was used in place of the anti-CD19 scFv domain was prepared.

### Test Example 1-7: Preparation of CD37.28z.SH Construct

In the same manner as in Test Example 1-1, a CAR construct that is the same as the CD19.28z.SH construct except that an anti-CD37 scFv domain (amino acid sequence: SEQ ID NO: 25; base sequence: SEQ ID NO: 26) was used in place of the anti-CD19 scFv domain was prepared.

### Test Example 1-8: Preparation of CD37.BBz.SH Construct

In the same manner as in Test Example 1-1, a CAR construct that is the same as the CD19.BBz.SH construct except that an anti-CD37 scFv domain (amino acid sequence: SEQ ID NO: 25; base sequence: SEQ ID NO: 26) was used in place of the anti-CD19 scFv domain was prepared.

### Test Example 1-9: Preparation of CD20.CD79a.40z.SH Construct

In the same manner as in Test Example 1-1, a CAR construct that is the same as the CD19.CD79a.40z.SH construct except that an anti-CD20 scFv domain (amino acid sequence: SEQ ID NO: 27; base sequence: SEQ ID NO: 28) was used in place of the anti-CD19 scFv domain was prepared.

### Test Example 1-10: Preparation of CD20.28z.SH Construct

In the same manner as in Test Example 1-1, a CAR construct that is the same as the CD19.28z.SH construct except that an anti-CD 20 scFv domain (amino acid sequence: SEQ ID NO: 27; base sequence: SEQ ID NO: 28) was used in place of the anti-CD19 scFv domain was prepared.

### Test Example 1-11: Preparation of CD20.BBz.SH Construct

In the same manner as in Test Example 1-1, a CAR construct that is the same as the CD19.BBz.SH construct except that an anti-CD20 scFv domain (amino acid sequence: SEQ ID NO: 27; base sequence: SEQ ID NO: 28) was used in place of the anti-CD19 scFv domain was prepared.

### Test Example 2: Preparation of CAR-T Cells

CAR-T cells were prepared using the CAR constructs of Test Example 1. Specifically, CAR-T cells were prepared as follows.

A gammaretroviral supernatant was prepared using a Phoenix-Ampho system (Orbigen). PBMCs were isolated by centrifugation of whole blood from a healthy donor using Ficoll-Paque (GE Healthcare). Subsequently, CD3⁺ lymphocytes were purified with immunomagnetic beads (Miltenyi Biotec) and activated with anti-CD3/CD28 beads (Invitrogen) at a 1:1 ratio. Retroviral transduction was performed on days 3 and 4 using a recombinant human RetroNectin fragment-coated plate (RetroNectin, Takara Bio). The retroviral supernatant was added to the RetroNectin fragment-coated plate, followed by centrifugation at 2100 rpm and 32°C for 1 hour. Thereafter, the virus liquid was removed from the plate, and then the CD3⁺ lymphocytes were added to the wells for gene transfer. The transformed T cells were cultured in RPMI 1640 medium (culture medium, CM) containing 10% human serum, 0.8 mM L-glutamine, 1% penicillin-streptomycin, and 0.5 µM 2-mercaptoethanol, and 50 IU/mL recombinant human IL-2 was added. On day 10, CAR-positive T cells were concentrated with biotin-labeled anti-EGFR mAb and streptavidin beads (Miltenyi Biotec). The concentrated CAR-positive T cells were grown by performing culture using γ-irradiated EBV-LCL in CM at a responder/stimulator ratio of 1:7 and adding 50 IU/mL IL-2 on days 1, 4 and 7. For the T-cell phenotype, EBV-LCL expanded CAR-T cells were stimulated with γ-irradiated CD19-K562 cells at a ratio of 1:1 for 4 consecutive weeks. For mouse experiments, the concentrated CAR-positive cells were stimulated a second time with anti-CD3/CD28 beads at a ratio of 1:1 for 7 to 10 days before use. Untransduced T cells were cultured in parallel according to the same procedure used for the transduced T cells, except for the addition of virus.

### Test Example 3: Evaluation 1 of Anticancer Effect

The anticancer effect of the CAR-T cells (into which the construct of Test Example 1-1, Test Example 1-2, or Test Example 1-3 was introduced) prepared in Test Example 2 was evaluated. Specifically, the evaluation was performed as follows.

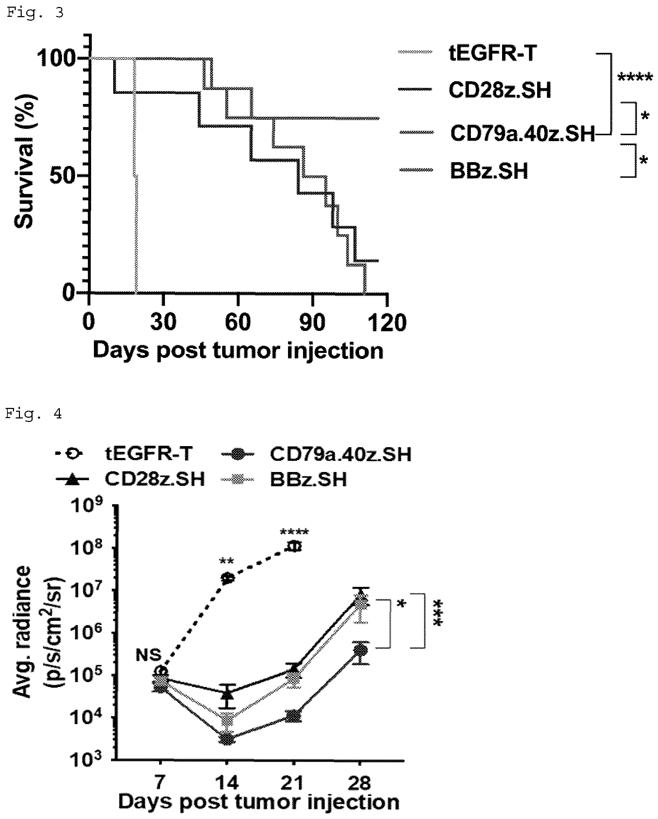

6- to 8-week-old male NSG (NOD-SCID common gamma chain knock out) mice were inoculated intravenously via the tail vein with 0.5×10⁶ Raji-ffluc cells. One week later, the mice were treated by intravenous administration of 1×10⁶ cells having tEGFR introduced therein or CD19 CAR-T cells (Test Example 2) via the tail vein. Bioluminescence imaging (BLI) was performed to assess tumor progression using an IVIS spectrum system. Fig. 2 shows the results of BLI, and Fig. 3 shows survival curves.

Figs. 2 and 3 show that the anticancer effect of the CD19.CD79a.40z.SH-transduced CAR-T cells was higher than those of the CD19.28z.SH-transduced CAR-T cells and the CD19.BBz.SH-transduced CAR-T cells.

### Test Example 4: Evaluation 2 of Anticancer Effect

The anticancer effect of the CAR-T cells (into which the construct of Test Example 1-1, Test Example 1-2, Test Example 1-3, Test Example 1-4, or Test Example 1-5 was introduced) prepared in Test Example 2 was evaluated. Specifically, the evaluation was performed as follows.

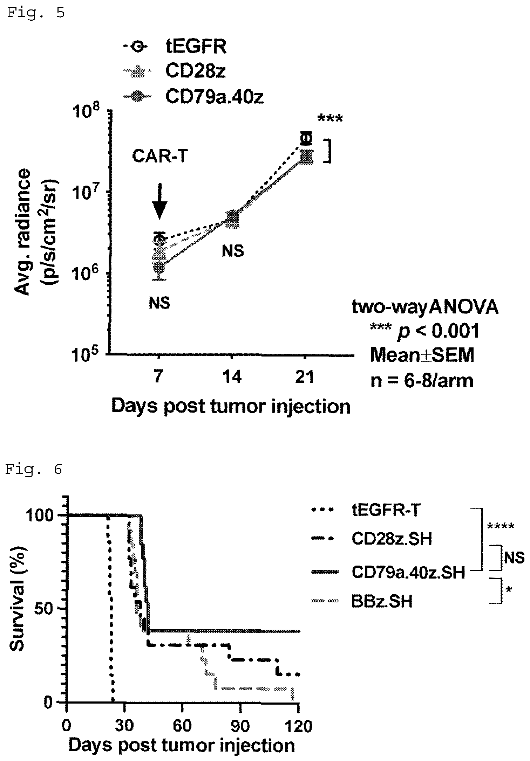

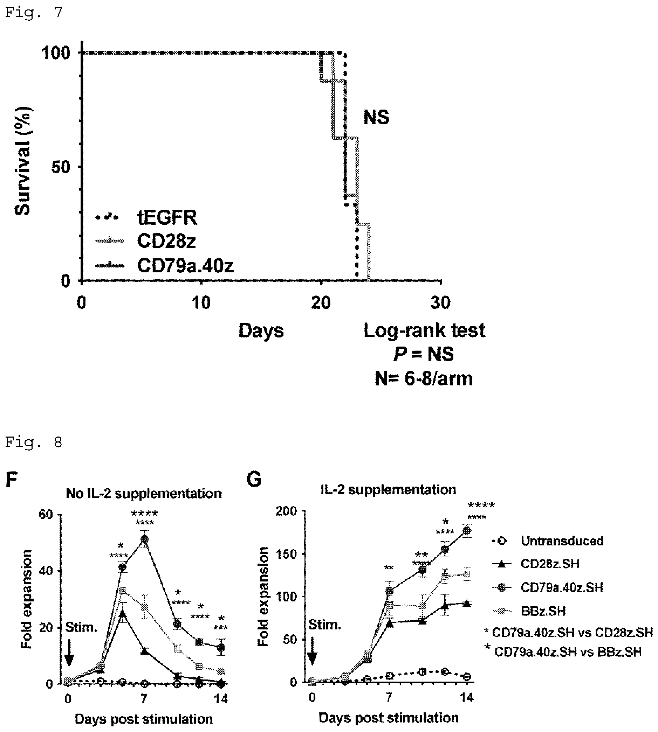

6- to 8-week-old male NSG mice were inoculated intravenously via the tail vein with 5×10⁵ NALM6 cells having the firefly luciferase gene introduced therein. One week later, the mice were treated by intravenous administration of 2×10⁶ cells having tEGFR introduced therein or CD19 CAR-T cells (Test Example 2) via the tail vein. Bioluminescence imaging (BLI) was performed to assess tumor progression using an IVIS spectrum system. Figs. 4 and 5 show the results of BLI, and Figs. 6 and 7 show survival curves. Figs. 4 and 6 show the results of CAR-T cells transduced with short-hinge constructs (constructs of Test Example 1-1, Test Example 1-2, and Test Example 1-3), and Figs. 5 and 7 show the results of CAR-T cells transduced with long-hinge constructs (constructs of Test Example 1-4 and Test Example 1-5).

Figs. 4 to 7 showed that the anticancer effect was higher in the case of using the short-hinge constructs. In particular, the CD79a.40z.SH-transduced CAR-T cells had a high anticancer effect.

### Test Example 5: Evaluation 3 of Anticancer Effect

The anticancer effect of the CAR-T cells (into which the construct of Test Example 1-1, Test Example 1-2, or Test Example 1-3 was introduced) prepared in Test Example 2 was evaluated. Specifically, the CAR-T cells and K562 (irradiated antigen-positive cells) having the CD37 gene introduced therein were mixed at a ratio of 1:1 (CAR-T cells:K562 cells) and co-cultured. The culture was performed with or without 50 U/ml of IL-2 on days 1, 4, 7, and 11, and the number of viable cells was counted. Fig. 8 shows the results.

Fig. 8 shows that the CAR-T cell proliferation after antigen stimulation of the CD19.CD79a.40z.SH-transduced CAR-T cells was greater than those after antigen stimulation of the CD19.28z.SH-transduced CAR-T cells and CD19.BBz.SH-transduced CAR-T cells, both with and without IL-2.

### Test Example 6: Evaluation 4 of Anticancer Effect

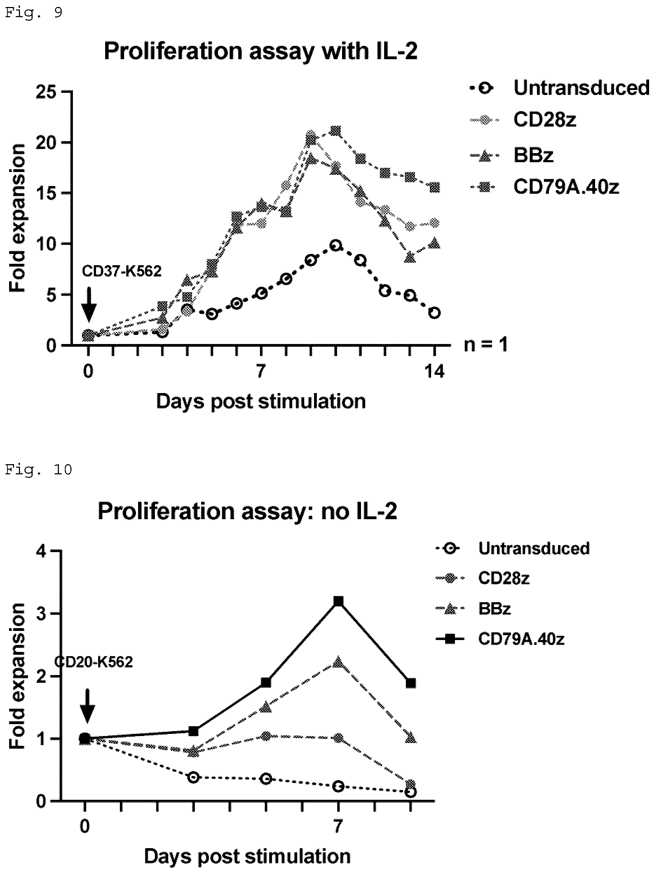

The anticancer effect of the CAR-T cells (into which the construct of Test Example 1-6, Test Example 1-7, or Test Example 1-8 was introduced) prepared in Test Example 2 was evaluated. Specifically, the CAR-T cells and K562 (irradiated antigen-positive cells) having the CD37 gene introduced therein were mixed at a ratio of 1:1 (CAR-T cells:K562 cells) and co-cultured. The culture was performed by adding 50 U/ml of IL-2 on days 1, 4, 7, and 11, and the number of viable cells was counted. Fig. 9 shows the results.

Fig. 9 shows that the CAR-T cell proliferation after antigen stimulation of the CD37.CD79a.40z.SH-transduced CAR-T cells was greater than those after antigen stimulation of the CD37.28z.SH-transduced CAR-T cells and CD37.BBz.SH-transduced CAR-T cells.

### Test Example 7: Evaluation 5 of Anticancer Effect

The anticancer effect of the CAR-T cells (into which the construct of Test Example 1-9, Test Example 1-10, or Test Example 1-11 was introduced) prepared in Test Example 2 was evaluated. Specifically, the CAR-T cells and K562 (irradiated antigen-positive cells) having the CD20 gene introduced therein were mixed at a ratio of 1:1 (CAR-T cells:K562 cells) and co-cultured without the addition of IL-2, and the number of viable cells was counted. Fig. 10 shows the results.

Fig. 10 shows that the CAR-T cell proliferation after antigen stimulation of the CD20.CD79a.40z.SH-transduced CAR-T cells was greater than those after antigen stimulation of the CD20.28z.SH-transduced CAR-T cells and the CD20.BBz.SH-transduced CAR-T cells.

## Claims

1. A chimeric antigen receptor, comprising an antigen-binding domain, a transmembrane domain, and an intracellular signal domain containing a CD79A intracellular domain and a CD40 intracellular domain,
wherein (A) the number of constituent amino acid residues between the antigen-binding domain and the transmembrane domain is 100 or less and/or (B) a target antigen of the antigen-binding domain is an antigen other than CD19.

2. The chimeric antigen receptor according to claim 1, wherein the intracellular signal domain contains an intracellular activation domain.

3. The chimeric antigen receptor according to claim 2, wherein the intracellular activation domain is at least one member selected from the group consisting of a CD3ζ intracellular domain and a FcεRIγ intracellular domain.

4. The chimeric antigen receptor according to claim 2 or 3, wherein the CD79A intracellular domain, the CD40 intracellular domain, and the intracellular activation domain are arranged in this order from the transmembrane domain side.

5. The chimeric antigen receptor according to any one of claims 1 to 4, comprising a hinge region of IgG or a portion thereof between the antigen-binding domain and the transmembrane domain.

6. The chimeric antigen receptor according to any one of claims 1 to 5, wherein the number of the constituent amino acid residues is 1 to 30.

7. The chimeric antigen receptor according to any one of claims 1 to 6, wherein the structure of the antigen-binding domain is a single-chain antibody structure.

8. The chimeric antigen receptor according to any one of claims 1 to 7, wherein the target antigen of the antigen-binding domain is at least one member selected from the group consisting of CD19, GD2, GD3, CD20, CD37, CEA, HER2, EGFR, type III mutant EGFR, CD38, BCMA, MUC-1, PSMA, WT1, cancer testis antigens, mutation peptides, hTERT, PRAME, TYRP1, mesothelin, PMEL, and mucin.

9. A polynucleotide encoding the antigen receptor according to any one of claims 1 to 8.

10. A cell comprising the polynucleotide according to claim 9.

11. The cell according to claim 10, which is a lymphocyte cell.

12. A pharmaceutical composition comprising the cell according to claim 10 or 11.

13. The pharmaceutical composition according to claim 12, which is for use in the treatment, prevention, or improvement of cancer.
